Europäisches Patentamt

(19) European Patent Office

Office européen des brevets

(11) Publication number: **0 079 478**
A1

(12) **EUROPEAN PATENT APPLICATION**

(21) Application number: 82109711.0

(51) Int. Cl.³: **A 61 M 15/00**

(22) Date of filing: 21.10.82

(30) Priority: 03.11.81 US 317837

(43) Date of publication of application:
25.05.83 Bulletin 83/21

(84) Designated Contracting States:
DE FR GB IT

(71) Applicant: MILES LABORATORIES INC.
1127 Myrtle Street
Elkhart Indiana 46514(US)

(72) Inventor: Elliott, Roderick Douglas
102 Stomp Road
Burnham Slough, SL1 7LX(GB)

(72) Inventor: Gardiner, Phillip John
55 Mayhew Crescent
Totteridge High Wycombe, HP13 6DF(GB)

(74) Representative: Jesse, Ralf-Rüdiger, Dr. et al,
Bayer AG Zentralbereich Patente Marken und Lizenzen
D-5090 Leverkusen 1 Bayerwerk(DE)

(54) Medicament inhalation device.

(57) This invention relates to an inhalation device (10) for dispensing finely-divided medicament powder compositions. The device has a medicament chamber (11) for storing finely-divided powder, an air inhalation chamber (12) having an air inlet (31,32) and air outlet mouthpiece (21), and a metered dose medicament delivery member (13,37,24). Medicament can pass from the medicament chamber (11) into the delivery member and is placed into the air inhalation chamber (12). Inhalation of air through the air inlet (31,32) effects movement of the medicament through the air inhalation chamber (12) and out the mouthpiece (21) into the respiratory tract.

FIG. 2

EP 0 079 478 A1

Croydon Printing Company Ltd

## MEDICAMENT INHALATION DEVICE

### BACKGROUND AND PRIOR ART

Hypersensitive individuals undergo an altered state as a result of contact with the antigens from an allergen, leading to the formation of antibodies to the allergen. Subsequent contact with one of those antigens or a structurally similar substance can evoke in an allergic individual a pathological reaction, due to the presence of such antibodies. A prominent manifestation includes bronchial asthma, characterized by obstruction of the lung airway passages.

In order to reduce these undesirable allergic responses, various medicaments which have an anti-allergic characteristic have been administered to patients.

It has been firmly established that the optimal route of medicament administration to provide therapeutic relief against such allergic response is by inhalation, which provides local administration to the lung airway lumen. Inhalation provides convenience and speed of action and often requires only a small dose of the pharmacologially active agent.

One type of inhalation device involves the use of conventional pressurized aerosol canisters. Some patients, however, find it difficult to synchronize inhalation through the mouth with activation of the

MS-1215

canister. Substances used as propellants in such aerosols may include fluorocarbons, which are safe when inhaled in small quantities, but may predispose individuals to ventricular arrhythmias if inhaler usage is excessive.

Various inhalation devices have been proposed and used which are capable of delivering into the patients' lungs medicaments which are dry powders. Typical of such inhalation devices are those described in U.S. Patent Nos. 3,507,277; 3,635,219, 3,669,113 and 3,870,046.

Inhalation devices of this type generally rely upon the motion of inhaled air to cause dispersion of the medicament into the air being inhaled. Many of the prior art devices are susceptible to clogging and are difficult to operate. Drug delivery by inhalation requires a device which is easy to use by young and older individuals, compact in size, and capable of delivering a metered dose of various sized particles. There is a need in the allergy field for such a simplified and efficient inhalation device.

## SUMMARY OF THE INVENTION

The present invention is a inhalation device comprising a medicament chamber capable of storing finely-divided medicament, an air inhalation chamber and a metered dose medicament delivery member, said medicament chamber having a downwardly directed funnel-shaped bottom wall having a bottom opening and a removable top-closure member, said air inhalation chamber having a top opening, side air inlet means and a mouthpiece air outlet means located opposite to said air inlet means, said medicament delivery member having a cavity of pre-determined volume, said cavity opening being alignable to sequentially communicate both with said bottom opening of said medicament chamber and said top opening of said air inhalation chamber, whereby when said cavity opening of said medicament delivery member is aligned with said bottom opening of said medicament chamber, finely-divided medicament in said medicament chamber can pass into and fill said cavity to provide a predetermined dose of said medicament, and when said cavity opening is aligned with the top opening of said air inhalation chamber, the predetermined dose of said medicament is metered into said air inhalation chamber, and subsequent inhalation of air from said air inlet means to said mouthpiece outlet means effects movement of said medicament through said inhalation chamber and out said mouthpiece for passage into the respiratory tract.

## DESCRIPTION OF THE DRAWINGS

Figure 1 is a front elevational view of one embodiment of a medicament inhalation device of the present invention;

Figure 2 is a vertical right side cross-sectional view of the medicament inhalation device taken along line 2-2 of Figure 1;

Figure 3 is a rear elevational view of the medicament inhalation device taken along line 3-3 of Figure 2 showing one embodiment of the air inlet system;

Figure 4 is the same view as in Figure 3 showing a modified embodiment of the air inlet system;

Figure 5 is a vertical rear cross-sectional view of the medicament inhalation device taken along line 5-5 of Figure 2;

DESCRIPTION OF THE INVENTION

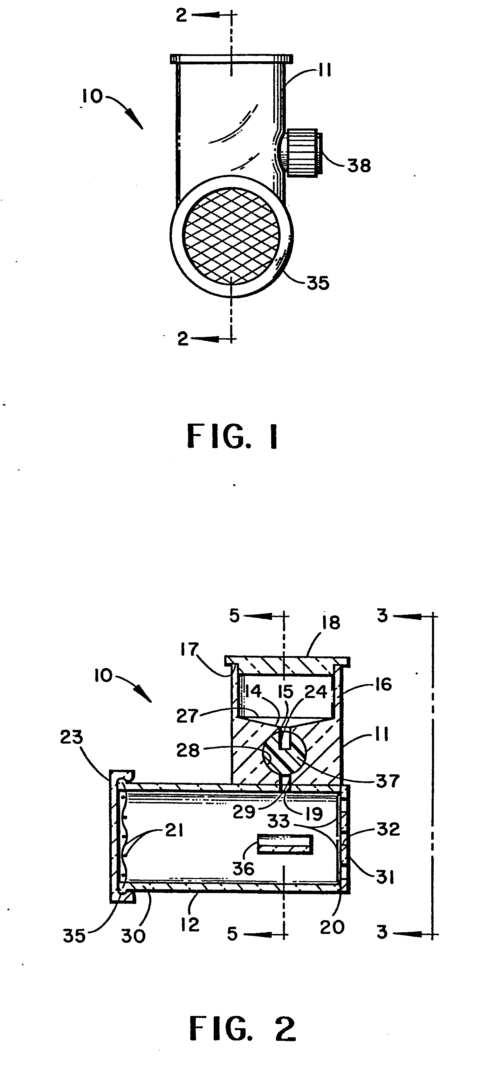

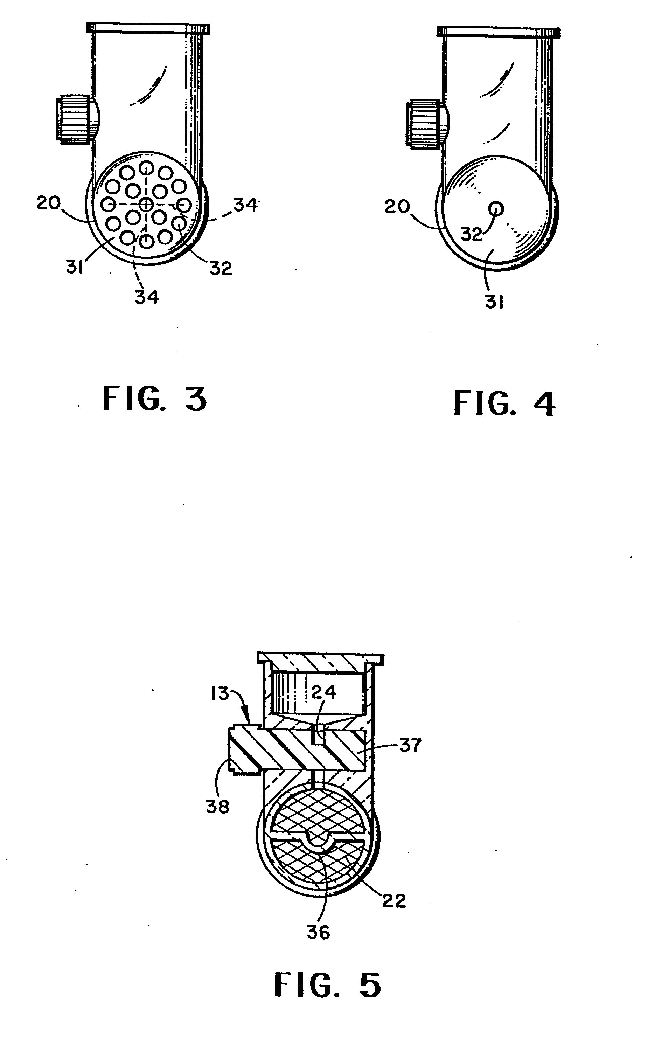

Referring to FIGS. 1-5, the illustrated embodiment of the present invention comprises a medicament inhalation device 10 having a medicament chamber 11, an air inhalation chamber 12, and a metered dose medicament delivery member 13. Medicament chamber 11 is generally vertically cylindrical in shape and is formed with sidewalls 16 and bottom wall surface 27 having a downwardly directed funnel-shaped bottom wall portion 14 formed therein having a bottom opening 15 which communicates with a vertical passage 29. Medicament chamber 11 also has a transverse horizontal cylindrical passage 28 formed therein communicating with passage 29 and bottom opening 15. Sidewalls 16 terminate in an upper edge surface 17 which has a removable closure member 18 associated therewith. Air inhalation chamber 12 is generally horizontally cylindrical in shape and has sidewalls 30 with a top opening 19 formed therein communicating with passage 29. One end 20 of chamber 12 is closed with an air inlet means comprising an inlet element 31 having a plurality of inlet openings 32 therein and a flexible diaphragm 33 behind element 31, having central slits 34 therein (FIGS. 2 and 3). The other end 21 of chamber 12 forms a mouthpiece air outlet means. End 21 has a peripheral rim 35 and a gauze 22 extending across the opening thereof (FIG. 5). End 21 can also have a removable protective cap 23 positioned thereon. FIG. 5 shows that chamber 12 also has a medicament collector 36 located beneath opening 19.

The metered dose medicament delivery member 13 comprises a cylindrical solid shaft 37 rotatably positioned within passage 28. Member 13 has a cavity 24 formed therein having a cross-sectional diameter the same as that of passage 29 and having a pre-determined volume. Member 13 has a knurled knob 38 attached thereto and which extends exterior to chamber 11.

FIG. 3 shows the slits 34 of diaphragm 33 which are located perpendicular to each other. FIG. 4 shows a further embodiment wherein end 20 is sealed with member 31 having a single central inlet opening 32.

In order to utilize the device of this invention, closure member 18 is removed from medicament chamber 11 and a desired amount of finely-divided medicament, for example, 40 to 50 mg of a powder containing an active anti-allergic component in combination with a pharmaceutically acceptable carrier, is placed in the medicament chamber 11. The finely-divided medicament can range from about 0.5 to 5 microns in particle size as a preferred range for efficient administration to the lung airway lumen. The cavity opening 24 of metered dose medicament delivery member 13 is then aligned with bottom opening 15 of medicament chamber 11 to allow a pre-determined dose of medicament powder to pass into cavity opening 24. Rotation of member 13 to the position shown in FIG. 2 produces sequential alignment of cavity opening 24 with top opening 19 of air inhalation chamber 12. This meters the pre-determined dose of medicament powder contained in cavity opening 24 into air inhalation chamber 12. Mouthpiece protective cap 23 is removed from mouthpiece air outlet means 21, and subsequent inhalation of air from air inlet means 20 effects movement of the medicament through the air inhalation chamber 12 and out mouthpiece air outlet means 21 for passage into the respiratory tract.

Medicament chamber 11 air inhalation chamber 12 and delivery member 13 are preferably formed from suitable rigid organoplastic materials, such as poly-

ethylene, polypropylene, polystyrene, styrene-acrylo-nitrile copolymers, polycarbonate, acetal copolymers, nitrile-acrylonitrile-styrene copolymers, polymethyl-pentene, polyacrylate and polymethacrylate and the like. For ease of visual viewing of the amount of medicament passing through device 10, chambers 11 and 12 are desirably optically transparent. Gauze 22 is preferably of fine stainless steel, having a mesh opening slightly larger than the largest particle size medicament to be administered to prevent any undesirably large particle size medicament from being inhaled into the respiratory tract.

WHAT IS CLAIMED IS:

1. An inhalation device comprising in combination a medicament chamber capable of storing finely-divided medicament therein, an air inhalation chamber and a metered dose medicament delivery member, said medicament chamber having a downwardly directed funnel-shaped bottom wall having a bottom opening therein and having sidewall means extending upwardly from said bottom wall to define said chamber therewith, said sidewall means terminating in an upper edge surface having a closure member removably positioned therewith, said air inhalation chamber having a top opening therein, a side air inlet means and a mouthpiece air outlet means located opposite to said air inlet means, said medicament delivery member having a cavity of predetermined volume with an opening therefor, said cavity opening being alignable to sequentially communicate both with said bottom opening of said medicament chamber and said top opening of said air inhalation chamber, whereby when said cavity opening of said medicament delivery member is aligned with said bottom opening of said medicament chamber, any finely-divided medicament in said medicament chamber can pass into and fill said cavity opening of said medicament delivery member to provide a predetermined dose of said medicament, and when said cavity opening is aligned with said top opening of said air inhalation chamber, the predetermined dose of said medicament is metered into said air inhalation chamber, subsequent inhalation of air from said air inlet means to said mouthpiece air outlet means effects movement

(claim 1 continued)

of said medicament through said air inhalation chamber and out said mouthpiece for passage into the respiratory tract.

2.  An inhalation device according to claim 1 wherein said air inlet means comprises a diaphragm air inlet system.

3.  An inhalation device according to claim 1 or 2 wherein said air inlet means comprises an opening in said air inhalation chamber.

4.  An inhalation device according to claims 1 to 3 wherein said air inhalation chamber contains collection means for ensuring collection of said finely-divided medicament prior to inhaling air into said air chamber.

5.  An inhalation device according to claims 1 to 4 wherein said mouthpiece is provided with a removable protective cap.

FIG. I

FIG. 2

FIG. 3

FIG. 4

FIG. 5

| | DOCUMENTS CONSIDERED TO BE RELEVANT | | EP 82109711.0 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| X | US – A – 2 587 215 (F.P. PRIESTLY)<br>* Fig. 3,4 and especially Fig. 16; column 4, lines 22-51; column 6, lines 26-68 *<br>-- | 1,3 | A 61 M 15/00 |
| A | US – A – 4 200 099 (GUENZEL)<br>* Fig. 1-4; column 2, line 21 – column 3, line 43 *<br>-- | 1,3,4 | |
| A | US – A – 4 274 403 (R.L. STRUVE)<br>* Totality *<br>-- | 1 | |
| A | DE – C – 323 610 (E. ENGEL)<br>* Totality *<br>---- | 1,3 | |
| | | | **TECHNICAL FIELDS SEARCHED (Int. Cl. ³)**<br><br>A 61 M 15/00<br>A 61 M 11/00 |

The present search report has been drawn up for all claims

| Place of search | Date of completion of the search | Examiner |
|---|---|---|
| VIENNA | 18-02-1983 | LUDWIG |